(19) Europäisches Patentamt
European Patent Office
Office européen des brevets

(11) Publication number: **0 128 338**
**B1**

(12) **EUROPEAN PATENT SPECIFICATION**

(45) Date of publication of patent specification: **05.08.87**

(51) Int. Cl.[4]: **C 07 D 407/06,** A 61 K 31/35

(21) Application number: **84105070.1**

(22) Date of filing: **04.05.84**

(54) **Silver pseudomonate, compositions containing it and its use in treating pseudomonal infections.**

(30) Priority: **12.05.83 GB 8313035**

(43) Date of publication of application:
**19.12.84 Bulletin 84/51**

(45) Publication of the grant of the patent:
**05.08.87 Bulletin 87/32**

(84) Designated Contracting States:
**BE CH DE FR GB IT LI NL SE**

(56) References cited:
**EP-A-0 005 614**
**EP-A-0 068 680**
**GB-A-1 395 907**

(73) Proprietor: **BEECHAM GROUP PLC**
**Beecham House Great West Road**
**Brentford Middlesex TW8 9BD (GB)**

(72) Inventor: **Mansford, Keith Robert Leonard**
**"Ashcroft", 9 Cavendish Road**
**Redhill Surrey (GB)**

(74) Representative: **Russell, Brian John et al**
**European Patent Attorney Beecham**
**Pharmaceuticals Great Burgh Yew Tree Bottom Road**
**Epsom Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 128 338 B1

**Description**

The present invention relates to silver pseudomonate, compositions containing it and its use in treating pseudomonal infections.

Pseudomonic acid is an antibiotic produced by aerobically culturing *Pseudomonas fluorescens*. The compound, of formula (I) below, and its salts and esters are disclosed and claimed in UK Patent No. 1 395 907.

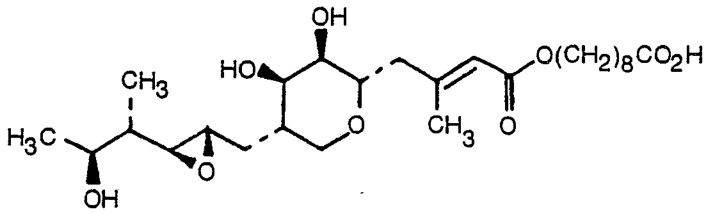

(1)

Whilst pseudomonic acid and its salts and esters are active against a variety of human and animal pathogens (see for instance UK Patent Nos. 1 577 730 and 1 577 545), they are not active at useful levels against *Pseudomonas* species.

*Pseudomonas* organisms tend to infect burns and wounds. Such infections are often difficult to treat as the organisms are not particularly susceptible to antibiotics.

It has now surprisingly been found that silver pseudomonate is active against *Pseudomonas* organisms, especially *Pseudomonas aeruginosa*, the causative agent of 'blue pus' infections.

The silver salt of pseudomonic acid has not been specifically disclosed in the above patents or any other publications and is, therefore, novel.

Accordingly the present invention provides, in one aspect, silver pseudomonate.

The invention also provides silver pseudomonate for use in the treatment of the human or animal body.

Apart from its surprising activity against *Pseudomonas*, silver pseudomonate has a similar spectrum of activity against pathogens to those of pseudomonic acid and sodium pseudomonate.

Accordingly the present invention also provides silver pseudomonate for use in treating the human or animal body, especially for teating infected wounds and burns.

The invention also provides a process for producing silver pseudomonate which process comprises reacting silver ions and pseudomonic acid or pseudomonate ions in aqueous solution and thereafter recovering the silver pseudomonate so formed.

Suitably the process is effected by adding a source of silver ions to an aqueous solution of pseudomonic acid or a pseudomonate salt, especially sodium pseudomonate.

Suitably the solution of pseudomonic acid or pseudomonate ions is the product of aerobically culturing *Pseudomonas fluorescens* (NCIB 10586). Such a solution may be the culture medium in which the organisms have been grown or it may have been produced by purifying such a medium for instance by extracting pseudomonic acid from such a culture medium using a polar, organic, water-immiscible solvent as described in EP 0 005 614. Alternatively the solution of pseudomonic acid or pseudomonate ions may be produced by dissolving pseudomonic acid or preferably a salt thereof, in an aqueous solvent. Preferably the solution is produced by dissolving pure sodium pseudomonate in water.

The source of silver ions is preferably a soluble silver salt such as silver nitrate or silver carbonate.

The invention further provides silver pseudomonate in substantially pure form, preferably at least 75% pure, more preferably at least 90% pure, most preferably at least 95% pure.

If precipitated from solution containing solvents other than water, the silver pseudomonate may be produced in a solvated form. If precipitated from aqueous solution the silver pseudomonate may be in a hydrated form.

Accordingly the invention further provides solvated, including hydrated, silver pseudomonate.

Silver pseudomonate may be administered as the pure compound (hereinafter referred to as the 'drug') or it may be administered as a pharmaceutical composition in association with a suitable carrier.

Accordingly the invention also provides a pharmaceutical formulation comprising silver pseudomonate and a pharmaceutically acceptable carrier therefor.

As used herein the term 'pharmaceutically acceptable' includes 'veterinarily acceptable'.

The formulations may be adapted for administration by any route, and would depend on the disease being treated. Normally, the formulations will be presented as topical solutions or suspensions for application to the skin, ears or eyes. Alternatively the formulations may be dry powders for application as an aerosol, or they may be presented as impregnated dressings for wounds and burns.

For topical application to the skin the drug may be made up into a cream, lotion or ointment. Cream or ointment formulations that may be used for the drug are conventional formulations well known in the art, for example, as described in standard text books of pharmaceutics and cosmetics, such as Harry's Cosmeticology published by Leonard Hill Books, and the British Pharmacopoeia. Alternatively the drug may be applied as a dry powder from an aerosol using conventional diluents and propellants.

For topical application to the ear, the drug may be made up into a solution or suspension in a suitable liquid carrier, such as water, glycerol, diluted ethanol, propylene glycol, polyethylene glycol or fixed oils.

For topical application to the eye, the drug is formulated as a solution or suspension in a suitable, sterile aqueous or non-aqueous vehicle. Additives, for instance buffers such as sodium metabisulphite or disodium edetate; preservatives including bactericidal and fungicidal agents, such as phenylmercuric acetate or nitrate or chlorhexidine, and thickening agents such as hypromellose may also be included.

Particularly suitable topical formulations comprise silver pseudomonate and at least 1% by weight of a poly(substituted or unsubstituted alkylene) glycol or a derivative thereof.

As used herein the term 'poly (substituted or unsubstituted alkylene) glycol' refers to polymers having the following repeating unit

$$-(CH_2)_nO-$$

wherein n is an integer, preferably 2 or 3 and to such polymers wherein one or more methylene groups of each repeating unit is substituted. Suitable substituents include alkoxy groups such as methoxy as in polymethoxypropylene glycol. Such polymers are known by a variety of names, for instance when n = 2, as polyethylene glycol, polyoxyethylene, polyoxyethylene glycol and macrogol and, when n = 3, as polypropylene glycol, polyoxypropylene and polyoxypropylene glycol. All these are useful in the invention as are derivatives of these polymers.

Suitable derivatives include ethers and esters of the poly (substituted or unsubstituted alkylene) glycols, such as the macrogol ethers and esters, for instance cetomacrogol, glycofurol, the 'Tweens'* and block copolymers including poly (substituted or unsubstituted alkylene) glycols such as Poloxamers which are block copolymers of polyethylene glycol and polypropylene glycol for instance the 'Pluronics'*, and cross-linked polyethylene glycol.

The poly (substituted or unsubstituted alkylene) glycols and derivatives thereof may be used singly or various grades and types may be used in combination to achieve the desired physical properties of the formulation.

Preferably the formulation comprises polyethylene glycol or a derivative thereof.

Suitably the formulation comprises from 0.01 to 50% by weight of silver pseudomonate, preferably 0.1 to 25%, more preferably 0.5 to 10% and most preferably about 2% by weight of silver pseudomonate calculated as the free acid. Such formulations comprising only silver pseudomonate and a poly (substituted or unsubstituted alkylene) glycol or derivative thereof will, of course, contain up to 99.99% of the poly (substituted or unsubstituted alkylene) glycol or derivative thereof.

The formulation may comprise additional therapeutic agents such as antibacterial, antifungal, antiviral and antiinflammatory agents, for instance chlortetracycline, miconazole, idoxuridine and phenazone, provided that these are compatible with the silver pseudomonate. Silver Pseudomonate tends to undergo a rearrangement reaction in the presence of acid and accordingly acidic agents are unlikely to be compatible with silver pseudomonate.

In a particular aspect the invention provides a topical formulation as described above wherein silver pseudomonate is the sole therapeutic agent.

In another aspect the invention provides a topical formulation comprising silver pseudomonate and at least 1% by weight of polyethylene glycol or a derivative thereof.

Polyethylene glycols (PEG's) and derivatives thereof are commercially available in a variety of chain lengths and with a variety of consistencies, for instance:

Polyethylene Glycols:

| Liquids | Semisolids | Hard Solids |
|---|---|---|
| PEG 200 | PEG 1000 | PEG 4000* |
| PEG 300 | PEG 1540 | PEG 6000 |
| PEG 400 | | |

*'Tween' and 'Pluronic' are Trade Marks for the above types of polymer.

Polyethylene Glycol derivatives:

| Derivative | Chemical Composition | Consistency |
|---|---|---|
| Glycofurol | Tetrahydrofurfuryl alcohol polyethylene glycol ether | Liquid |
| Tween 60 | Polyoxyethylene Sorbitan monostearate | Semi-solid |
| Tween 80 | Polyoxyethylene Sorbitan monooleate | Liquid |

*PEG 4000 is the B.P. nomenclature for PEG with mean molecular weight of 3350. This material is also known as PEG 3350 in U.S.P. nomenclature.

These may be used singly or admixed in suitable proportions to achieve the desired consistency of formulation.

The formulations of the present invention may contain appropriate conventional additives such as preservatives, solvents to assist drug penetration and emollients in ointments and creams. The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Particularly suitable formulations according to the present invention comprise at least 1% by weight of PEG or a mixture of PEG's, from 0 to 25% by weight of a PEG derivative or mixture of PEG derivatives and from 0.5 to 10% by weight of silver pseudomonate calculated as the free acid.

Preferably the silver pseudomonate represents 1 to 5% of the formulation, most preferably about 2% of the formulation calculated as the free acid.

Formulations of the invention may be produced by conventional pharmaceutical techniques. Thus ointments and creams are conveniently prepared by melting and mixing together the solid or semi-solid PEG's or PEG analogues or derivatives, and stirring in the therapeutic agent and any other ingredients. The product is then slowly cooled and filled into containers such as collapsible metal or plastic tubes.

Liquid preparations, such as ear and eye drops, are produced by dissolving the therapeutic agent in the liquid PEG's or PEG analogues or derivatves and the other ingredients are then added. The resulting solution or suspension is distributed into glass or plastic bottles or in single dose packs such as soft gelatin capsules which are then heat sealed.

If necessary the formulation may be milled at any suitable stage of the process.

A suitable sterilisation procedure may be included in the above processes if necessary. Alternatively raw materials are obtained in sterile conditions and the formulations are produced aseptically.

The dosage employed for formulations administered topically will, of course, depend on the size of the area being treated. For the ears and eyes each dose will typically be in the range from 10 to 100 mg of the drug.

The present invention further provides a process for producing a pharmaceutical formulation which process comprises bringing into association silver pseudomonate and a pharmaceutically acceptable carrier therefor.

The invention will now be illustrated with reference to the following Examples and Biological data.

Example 1

Silver Pseudomonate A

Sodium pseudomonate A (1.82 g, 4 mmol) and silver nitrate (0.68 g, 4 mmol) were stirred in distilled water for 30 min resulting in the formulation of a white gelatinous precipitate. The mixture was centrifuged, the aqueous layer removed and the residue washed with distilled water. The suspension was centrifuged and the residual solid was dried over phosphorous pentoxide under high vacuum for 2 days to yield silver pseudomonate A, m.p. 164—166°C, (855 mg, 35%); $\gamma_{max}$(KBr) 3400, 1710, 1645, 1515 $cm^{-1}$; $\delta H(CD_3)_2SO)$ 5.68 (1H, s, H2), 2.12 (3H, s, $CH_3$—15), 1.1 (3H, d, $CH_3$—14), 0.85 (3H, d, $CH_3$—17) (Found: C, 49.6; H, 6.7; Ag, 17.8. $C_{26}H_{43}O_9Ag$ requires C, 51.4; H, 7.1; Ag, 17.8%).

Example 2

Liquid Formulation

Silver pseudomonate may be dissolved in PEG 400 and the formulation adjusted, by addition of further PEG 400, to contain 2% by weight of silver pseudomonate.

Example 3

Ointment Formulation

| | % w/w |
|---|---|
| PEG 400 | 59 |
| PEG 4000 | 39 |
| Silver pseudomonate | 2 |

The formulation may be produced by melting the mixture of PEG's and stirring in the silver pseudomonate.

Example 4

Lotion Formulation

| | % w/w |
|---|---|
| PEG 400 | 74 |
| Ethanol | 24 |
| Silver pseudomonate | 2 |

Example 5

Drop Formulation

| | % w/w |
|---|---|
| PEG 400 | 74 |
| Glycofurol | 24 |
| Silver pseudomonate | 2 |

Example 6

| | % w/w |
|---|---|
| Cetomacrogol emulsifying ointment | 65 |
| Polyethylene glycol 200 | 33 |
| Silver pseudomonate | 2 |

BIOLOGICAL DATA

a) The minimum inhibitory concentrations (MICs) of silver pseudomonate and sodium pseudomonate were determined against 20 strains of *Pseudomonas aeruginosa* in Blood Agar Base. Typical results are presented in Table 1. Silver pseudomonate was more active than sodium pseudomonate against all strains tested.

b) MIC's of silver and sodium pseudomonate against various pathogenic bacteria were determined by standard methods. Typical results are presented in Table 2.

Table 1

The activity of Sodium Pseudomonate and Silver Pseudomonate against 20 strains of Pseudomonas aeruginosa:

Typical MIC's

| Pseudomonas aeruginosa | MIC* ug/ml | |
|---|---|---|
| | Sodium Salt | Silver Salt |
| NCTC 10662 | 12,800 | 128 |
| Dalgleish | >128 | 128 |
| PU7 | >128 | 128 |
| W985 | >128 | 128 |
| S41 | >128 | 128 |
| R60 | >128 | 128 |
| Pu4 | >128 | 128 |
| R59 | >128 | 64 |
| T3 | >128 | 128 |
| R3 | 6,400 | 128 |
| R139 | >128 | 128 |
| R22 | >128 | 128 |
| W995 | >128 | 128 |
| 59 | >128 | 128 |
| 125 | >128 | 128 |
| 4 | >128 | 128 |
| Fr13 | 6,400 | 128 |
| D25 | >128 | 128 |
| ATCC 27853 | >128 | 128 |
| W996 | >128 | 128 |

*MIC determined in serial dilution in Blood Agar Base. Inoculum of 0.001 ml of an overnight Tryptone Soya Broth Culture. Incubated at 37°C overnight.

Table 2

Typical MIC's (μg/ml) against Human Bacteria

| Organism | Pseudomonate Salt, Silver | MIC (μg/ml) Sodium |
|---|---|---|
| E. coli NCTC 10418 | 128 | 125 |
| P. mirabilis 889 | 128 | 125 |
| K. aerogenes A | 128 | 250 |
| Ps. aeruginosa NCTC 10662 | 128 | 12800 |
| Pasteurella multocida 1633 | 0.5 | 0.25 |
| Haemophilus influenzae Wy21 | 0.12 | 0.12 |
| Bacillus subtilis 6633 | 0.25 | 0.25 |
| Corynebacterium xerosis 9755 | 128 | >125 |
| Staph. aureus Oxford | 0.5 | 0.25 |
| Staph. aureus Russell | 0.5 | 0.25 |
| Staph. aureus W2827 | 0.5 | 0.25 |
| Strep. faecalis I | 64 | 50 |
| Strep. pyrogenes R80/421—A | 0.25 | 0.25 |
| Strep. agalacitiae 2788—B | 1.0 | 0.5 |
| Strep. spp. 64/848—C | 1.0 | 0.5 |

**Claims**

1. The compound silver pseudomonate.
2. A compound as claimed in claim 1, being at least 75% pure.
3. A compound as claimed in claim 1 or claim 2, being at least 90% pure.
4. A compound as claimed in any one of claims 1 to 3, being at least 95% pure.
5. A compound as claimed in any one of claims 1 to 4 in the form of a solvate.
6. A compound as claimed in any one of claims 1 to 5 in the form of a hydrate.
7. A process for producing silver pseudomonate which process comprises reacting silver ions and pseudomonic acid or pseudomonate ions in aqueous solution and thereafter recovering the silver pseudomonate so formed.
8. A process as claimed in claim 7, effected by adding a source of silver ions to an aqueous solution of pseudomonic acid or a pseudomonate salt.
9. A process as claimed in claim 8, wherein the pseudomonate salt is sodium pseudomonate.
10. A process as claimed in claim 7 or claim 8, wherein the solution of pseudomonic acid or pseudomonate ions is the product of aerobically culturing *Pseudomonas fluorescens* (NCIB 10586).
11. A process as claimed in any one of claims 7 to 10, wherein the source of silver ions is a soluble silver salt.
12. A process as claimed in any one of claims 7 to 11, wherein the source of silver ions is silver nitrate.
13. A pharmaceutical formulation comprising silver pseudomonate and a pharmaceutically acceptable carrier therefor.
14. A pharmaceutical formulation as claimed in claim 13, formulated for topical application.

15. A pharmaceutical formulation as claimed in claim 13 or claim 14, comprising silver pseudomonate and at least 1% by weight of a poly (substituted or unsubstituted alkylene) glycol or a derivative thereof.

16. A pharmaceutical formulation as claimed in any one of claims 13 to 15, comprising polyethylene glycol or a derivative thereof.

17. A pharmaceutical formulation as claimed in any one of claims 13 to 16, comprising silver pseudomonate and at least 1% by weight of polyethylene glycol or a derivative thereof.

18. A pharmacuetical formulation as claimed in any one of claims 13 to 17, wherein silver pseudomonate is the sole therapeutic agent.

19. A compound as claimed in claim 1, for use in the treatment of the human or animal body infected with Pseudomonas organisms.

20. A compound as claimed in claim 19, for use in treating infected wounds and burns infected with Pseudomonas organisms.

**Patentansprüche**

1. Die Verbindung Pseudomonsäure-Silbersalz.
2. Eine Verbindung wie in Anspruch 1 beansprucht, welche wenigstens zu 75% rein ist.
3. Eine Verbindung wie in Anspruch 1 oder 2 beansprucht, welche mindestens zu 90% rein ist.
4. Eine Verbindung wie in irgendeinem der Ansprüche 1 bis 3 beansprucht, welche mindestens zu 95% rein ist.
5. Eine Verbindung wie in irgendeinem der Ansprüche 1 bis 4 beansprucht, in der Form eines Solvates.
6. Eine Verbindung wie in irgendeinem der Ansprüche 1 bis 5 beansprucht in der Form eines Hydrates.
7. Ein Verfahren zur Herstellung von Pseudomonsäure-Silbersalz, welches Verfahren das Umsetzen von Silberionen und Pseudomonsäure oder Pseudomonsäureionen in wäßriger Lösung und anschließend die Gewinnung des so gebildeten Pseudomonsäure-Silbersalzes umfaßt.
8. Ein Verfahren wie in Anspruch 7 beansprucht, welches durchgeführt wird durch Zusetzen einer Quelle für Silberionen zu einer wäßrigen Lösung von Pseudomonsäure oder eines Pseudomonsäuresalzes.
9. Ein Verfahren wie in Anspruch 8 beansprucht, in welchem das Pseudomonsäuresalz das Pseudomonsäure-Natriumsalz ist.
10. Ein Verfahren wie in Anspruch 7 oder Anspruch 8 beansprucht, in welchem die Lösung von Pseudomonsäure oder Pseudomonsäureionen das produkt der aerobischen Züchtung von *Pseudomonas fluorescens* (NCIB 10586) ist.
11. Ein Verfahren wie in irgendeinem der Ansprüche 7 bis 10 beansprucht, in welchem die Quelle für Silberionen ein lösliches Silbersalz ist.
12. Ein Verfahren wie in irgendeinem der Ansprüche 7 bis 11 beansprucht, in welchem die Quelle für Silberionen Silbernitrat ist.
13. Eine pharmazeutische Zusammensetzung, umfassend Pseudomonsäure-Silbersalz und einen dafür geeigneten pharmazeutisch annehmbaren Träger.
14. Eine pharmazeutische Zusammensetzung wie in Anspruch 13 beansprucht, welche abgestimmt ist für die topische Anwendung.
15. Eine pharmazeutische Zusammensetzung wie in Anspruch 13 oder Anspruch 14 beansprucht, umfassend Pseudomonsäure-Silbersalz und mindestens 1 Gewichtsprozent eines Poly(substituierten oder nicht-substituierten alkylen)glycols oder eines Derivates desselben.
16. Eine pharmazeutische Zusammensetzung wie in irgendeinem der Ansprüche 13 bis 15 beansprucht, umfassend Polyäthylenglykol oder ein Derivat desselben.
17. Eine pharmazeutische Zusammensetzung wie in igendeinem der Ansprüche 13 bis 16 beansprucht, umfassend Pseudomonsäure-Silbersalz und mindestens 1 Gewichtsprozent Polyäthylenglykol oder eines Derivates davon.
18. Eine pharmazeutische Zusammensetzung wie in irgendeinem der Ansprüche 13 bis 17 beansprucht, in welcher das Pseudomonsäure-Silbersalz der einzige therapeutische Wirkstoff ist.
19. Eine Verbindung wie in Anspruch 1 beansprucht, zur Anwendung für die Behandlung des mit Pseudomonas-Organismen infizierten menschlichen oder tierischen Körpers.
20. Eine Verbindung wie in Anspruch 19 beansprucht, zur Verwendung bei der Behandlung von mit Pseudomonas-Organismen infizierten Wunden und Brandwunden.

**Revendications**

1. Pseudomonate d'argent.
2. Composé suivant la revendication 1, qui est pur à 75% au moins.
3. Composé suivant la revendication 1 ou 2, qui est pur à 90% au moins.
4. Composé suivant l'une quelconque des revendications 1 à 3, qui est pur à 95% au moins.
5. Composé suivant l'une quelconque des revendications 1 à 4, sous la forme d'un solvat.
6. Composé suivant l'une quelconque des revendications 1 à 5, sous la forme d'un hydrate.

7. Procédé de production de pseudomonate d'argent, qui comprend la réaction d'ions argent et d'acide pseudomonique ou d'ions pseudomonate en solution aqueuse et ensuite la récupération du pseudomonate d'argent ainsi formé.

8. Procédé suivant la revendication 7, effectuée par addition d'une source d'ions argent à une solution aqueuse d'acide pseudomonique ou d'un sel pseudomonique.

9. Procédé suivant la revendication 8, dans lequel le sel pseudomonique est le pseudomonate de sodium.

10. Procédé suivant la revendication 7 ou la revendication 8, dans lequel la solution d'acide pseudomonique ou d'ions pseudomonate est le produit de la culture aérobie de Pseudomonas fluorescens (NCIB 10586).

11. Procédé suivant l'une quelconque des revendications 7 à 10, dans lequel la source d'ions argent est un sel d'argent soluble.

12. Procédé suivant l'une quelconque des revendications 7 à 11, dans lequel la source d'ions argent est le nitrate d'argent.

13. Formulation pharmaceutique comprenant du pseudomonate d'argent et un support convenable acceptable du point de vue pharmaceutique pour celui-ci.

14. Formulation pharmaceutique suivant la revendication 13, formulée pour l'application locale.

15. Formulation pharmaceutique suivant la revendication 13 ou 14, comprenant du pseudomonate d'argent et au moins 1% en poids d'un poly(alcoylène substitué ou non-substitué)glycol ou d'un dérivé de celui-ci.

16. Formulation pharmaceutique suivant l'une quelconque des revendications 13 à 15, comprenant du polyéthylèneglycol ou un dérivé de celui-ci.

17. Formulation pharmaceutique suivant l'une quelconque des revendications 13 à 16, comprenant du pseudomonate d'argent et au moins 1% en poids de polyéthylèneglycol ou d'un dérivé de celui-ci.

18. Formulation pharmaceutique suivant l'une quelconque des revendications 13 à 17, dans laquelle le pseudomonate d'argent est l'unique agent thérapeutique.

19. Composé suivant la revendication 1, utile dans le traitement du corps humain ou animal infecté avec des organismes de l'espèce Pseudomonas.

20. Composé suivant la revendication 19, utile dans le traitement de brûlures et de blessures infectées avec des organismes de l'espèce Pseudomonas.